(19) Europäisches Patentamt
European Patent Office
Office européen des brevets

(11) Veröffentlichungsnummer: **0 040 368**
**B1**

(12) # EUROPÄISCHE PATENTSCHRIFT

(45) Veröffentlichungstag der Patentschrift: **28.09.83**

(51) Int. Cl.³: **C 07 D 249/08**, A 01 N 43/64

(21) Anmeldenummer: **81103480.0**

(22) Anmeldetag: **07.05.81**

(54) **Triazolyl-vinyl-ketone und -carbinole, Verfahren zu ihrer Herstellung sowie ihre Verwendung als Fungizide.**

(30) Priorität: **19.05.80 DE 3019046**

(43) Veröffentlichungstag der Anmeldung: **25.11.81 Patentblatt 81/47**

(45) Bekanntmachung des Hinweises auf die Patenterteilung: **28.09.83 Patentblatt 83/39**

(84) Benannte Vertragsstaaten: **AT BE CH DE FR GB IT LI NL**

(56) Entgegenhaltungen:
**EP-A-0 015 387**
**EP-A-0 020 955**
**EP-A-0 022 975**
**DE-A-2 645 617**
**US-A-4 182 862**

**Die Akte enthält technische Angaben, die nach dem Eingang der Anmeldung eingereicht wurden und die nicht in dieser Patentschrift enthalten sind.**

(73) Patentinhaber: **BAYER AG, Zentralbereich Patente, Marken und Lizenzen, D-5090 Leverkusen 1, Bayerwerk (DE)**

(72) Erfinder: **Elbe, Hans-Ludwig, Dr., Dasnoeckel 59, D-5600 Wuppertal 11 (DE)**
Erfinder: **Büchel, Karl Heinz, Prof. Dr., Dabringhausener Strasse 42, D-5063 Burscheid (DE)**
Erfinder: **Frohberger, Paul-Ernst, Dr., Willi-Baumeister-Strasse 5, D-5090 Leverkusen (DE)**
Erfinder: **Brandes, Wilhelm, Dr., Eichendorffstrasse 3, D-5653 Leichlingen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

Triazolyl-vinyl-ketone und -carbinole, Verfahren zu ihrer Herstellung sowie ihre Verwendung als Fungizide

Die vorliegende Erfindung betrifft neue Triazolyl-vinylketone und -carbinole, ein Verfahren zu ihrer Herstellung sowie ihre Verwendung als Fungizide.

Es ist bereits bekanntgeworden, daß bestimmte 1-Phenyl-2-triazolyl-4,4-dimethyl-1-penten-3-one und -ole gute fungizide Wirksamkeit besitzen (vergleiche zum Beispiel die DE-OS 28 38 847). Die Wirkung dieser Verbindungen ist jedoch, insbesondere bei niedrigen Aufwandmengen und -konzentrationen, nicht immer ganz befriedigend.

Es wurden neue Triazolyl-vinyl-ketone und -carbinole der allgemeinen Formel

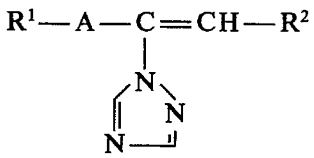

(I)

in welcher

A für die Ketogruppe oder eine CH(OH)-Gruppierung steht,

$R^1$ für Alkyl mit 1 bis 6 Kohlenstoffatomen oder für Halogenalkyl mit 1 bis 4 Kohlenstoffatomen und 1 bis 5 Halogenatomen steht,

$R^2$ für primäres oder tertiäres Alkyl mit 1 bis 29 Kohlenstoffatomen, für Halogenalkyl mit 1 bis 18 Kohlenstoff- und 1 bis 5 Halogenatomen steht, weiterhin für Alkoxyalkyl und Alkylmercaptoalkyl mit jeweils 1 bis 4 Kohlenstoffatomen in jedem Alkylteil steht, für Dialkylaminoalkyl mit 1 bis 4 Kohlenstoffatomen in den Alkylresten der Aminogruppe und 1 bis 18 Kohlenstoffatomen im Alkylteil steht, sodann für Hydroxyalkyl mit 1 bis 18 Kohlenstoffatomen steht, für Alkenyl, Alkinyl und Alkeninyl mit jeweils bis zu 6 Kohlenstoffatomen steht, wobei die drei letztgenannten Reste substituiert sein können durch Hydroxy, Alkoxy mit 1 bis 4 Kohlenstoffatomen und durch Phenyl, wobei letzteres selbst noch substituiert sein kann durch Halogen, durch Alkyl, Alkoxy und Alkylthio mit jeweils 1 bis 4 Kohlenstoffatomen und durch Halogenalkoxy mit 1 bis 4 Kohlenstoff- und 1 bis 5 Halogenatomen, und außerdem noch für Benzyl steht, welches im Phenylteil durch die weiter oben genannten Phenylsubstituenten und im Alkylteil durch Cyano substituiert sein kann, und schließlich noch für die Indenyl-, die Fluorenyl-, die Diphenylmethyl- und die Triphenylmethyl-Gruppe steht,

und deren physiologisch verträglichen Säureadditions-Salze sowie Metallsalz-Komplexe gefunden.

Die Verbindungen der Formel (I) können in zwei geometrischen Isomerenformen (E- und Z-Form) vorliegen, je nach Anordnung der Gruppen, die an die Doppelbindung gebunden sind; vorzugsweise fallen sie in einem wechselnden E,Z-Isomerenverhältnis an. Wenn A für die CH(OH)-Gruppierung steht, liegt ein asymmetrisches Kohlenstoffatom vor, so daß die Verbindungen der Formel (I) in diesem Fall außerdem in zwei optischen Isomerenformen anfallen; vorzugsweise fallen sie als Racemate an. Die vorliegende Erfindung betrifft sowohl die einzelnen Isomeren als auch die Isomeren-Gemische.

Weiterhin wurde gefunden, daß man die Triazolyl-vinylketone und -carbinole der Formel (I) erhält, wenn man Ketoenamine der Formel

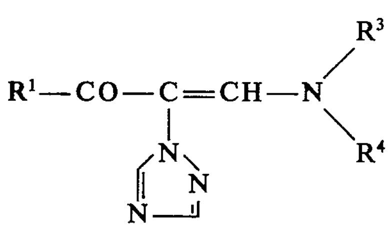

(II)

in welcher

$R^1$ die oben angegebene Bedeutung hat und

$R^3$ und $R^4$ gleich oder verschieden sind und für Alkyl mit 1 bis 4 Kohlenstoffatomen stehen,

mit magnesium-organischen Verbindungen der Formel

$$Hal-Mg-R^2 \quad (III)$$

in welcher

| | |
|---|---|
| $R^2$ | die oben angegebene Bedeutung hat und |
| Hal | für Halogen steht, |

in Gegenwart eines Lösungsmittels umsetzt, und gegebenenfalls die entstehenden Keto-Derivate der Formel (I) in üblicher Weise reduziert.

An die so erhaltenen Verbindungen der Formel (I) kann gegebenenfalls anschließend eine Säure oder ein Metallsalz addiert werden.

Die neuen Triazolyl-vinyl-ketone und -carbinole der Formel (I) weisen starke fungizide Eigenschaften auf. Dabei zeigen überraschenderweise die erfindungsgemäßen Verbindungen eine bessere fungizide Wirksamkeit als die aus dem Stand der Technik bekannten 1-Phenyl-2-triazolyl-4,4-dimethyl-1-penten-3-one und -ole, welche chemisch und wirkungsmäßig naheliegende Verbindungen sind. Die erfindungsgemäßen Stoffe stellen somit eine Bereicherung der Technik dar.

Die erfindungsgemäßen Triazolyl-vinyl-ketone und -carbinole sind durch die Formel (I) allgemein definiert.

Bevorzugt sind diejenigen Verbindungen der Formel (I), in denen $R^1$ für tert.-Butyl, Chlor-tert.-butyl, Fluor-tert.-butyl, Dichlor-tert.-butyl und Difluor-tert.-butyl steht.

Im einzelnen seien außer den bei den Herstellungsbeispielen genannten Verbindungen die folgenden Verbindungen der allgemeinen Formel (I) genannt:

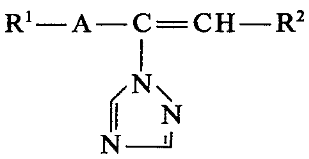

(I)

| $R^1$ | $R^2$ | A |
|---|---|---|
| $Cl—CH_2—C(CH_3)_2—$ | $—CH_2—$ 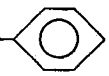 | CO |
| $Cl—CH_2—C(CH_3)_2—$ | $—C{\equiv}C—$ 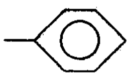 | CO |
| $Cl—CH_2—C(CH_3)_2—$ | $—C(CH_3)_3$ | CO |
| $F—CH_2—C(CH_3)_2—$ | $—CH_2—$ 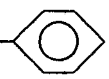 | CO |
| $F—CH_2—C(CH_3)_2—$ | $—C{\equiv}C—$ 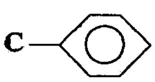 | CO |
| $F—CH_2—C(CH_3)_2—$ | $—C(CH_3)_3$ | CO |
| $CH_3—C(CH_2Cl)_2—$ | $—CH_3$ | CO |
| $CH_3—C(CH_2Cl)_2—$ | $—C_2H_5$ | CO |
| $CH_3—C(CH_2Cl)_2—$ | $—C(CH_3)_3$ | CO |
| $CH_3—C(CH_2Cl)_2—$ | $—C_6H_{13}$-n | CO |
| $CH_3—C(CH_2Cl)_2—$ | $—CH_2—$ 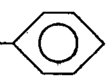 | CO |
| $CH_3—C(CH_2Cl)_2—$ | $—C{\equiv}C—$ 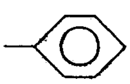 | CO |
| $CH_3—C(CH_2F)_2—$ | $—CH_3$ | CO |
| $CH_3—C(CH_2F)_2—$ | $—C_2H_5$ | CO |

Fortsetzung

| $R^1$ | $R^2$ | A |
|---|---|---|
| $CH_3—C(CH_2F)_2—$ | $—C(CH_3)_3$ | CO |
| $CH_3—C(CH_2F)_2—$ | $—C_6H_{13}$-n | CO |
| $CH_3—C(CH_2F)_2—$ | 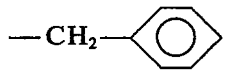 | CO |
| $CH_3—C(CH_2F)_2—$ | 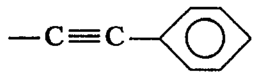 | CO |
| $(CH_3)_3C—$ | 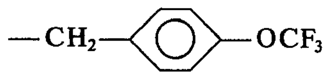 | CO |
| $(CH_3)_3C—$ | 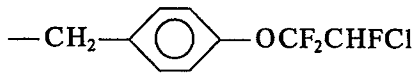 | CO |
| $(CH_3)_3C—$ | $—CH_2—CH_2—O—C_3H_7$-n | CO |
| $(CH_3)_3C—$ | $—C_{23}H_{47}$-n | CO |
| $(CH_3)_3C—$ | $—CH_2—CH_2—S—C_3H_7$-n | CO |
| $(CH_3)_3C—$ | $—(CH_3)_3—N(CH_3)_2$ | CO |
| $(CH_3)_3C—$ | $—(CH_2)_7—OH$ | CO |
| $(CH_3)_3C—$ | 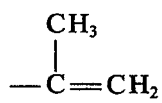 | CO |
| $(CH_3)_3C—$ | 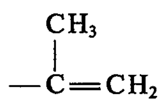 | CO |
| $(CH_3)_3C—$ | $—(CH_2)_7F$ | CO |
| $(CH_3)_3C—$ | $—(CH_2)_7Cl$ | CO |
| $(CH_3)_3C—$ | 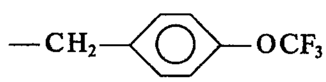 | CH(OH) |
| $(CH_3)_3C—$ | 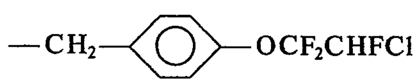 | CH(OH) |
| $(CH_3)_3C—$ | $—CH_2—CH_2—O—C_3H_7$-n | CH(OH) |
| $(CH_3)_3C—$ | $—CH_{23}H_{47}$-n | CH(OH) |
| $(CH_3)_3C—$ | $—CH_2—CH_2—S—C_3H_7$-n | CH(OH) |
| $(CH_3)_3C—$ | $—(CH_2)_3—N(CH_3)_2$ | CH(OH) |
| $(CH_3)_3C—$ | $—(CH_2)_7—OH$ | CH(OH) |
| $(CH_3)_3C—$ | 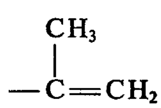 | CH(OH) |

Fortsetzung

| $R^1$ | $R^2$ | A |
|---|---|---|
| $(CH_3)_3C-$ | 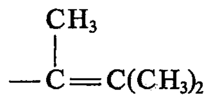 | CH(OH) |
| $(CH_3)_3C-$ | $-(CH_2)_7F$ | CH(OH) |
| $(CH_3)_3C-$ | $-(CH_2)_7Cl$ | CH(OH) |
| $(CH_3)_3C-$ | $-CH(CN)-$ 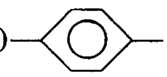 $-Cl$ | CH(OH) |
| $(CH_3)_3C-$ | $-C_6H_{13}$-n | CH(OH) |
| $(CH_3)_3C-$ | $-C_9H_{19}$-n | CH(OH) |
| $(CH_3)_3C-$ | $-C_{12}H_{25}$-n | CH(OH) |

Verwendet man beispielsweise 4,4-Dimethyl-1-dimethylamino-2-(1,2,4-triazol-1-yl)-1-penten-3-on und tert.-Butyl-magnesiumbromid als Ausgangsstoffe, so kann der Reaktionsablauf des erfindungsgemäßen Verfahrens durch das folgende Formelschema wiedergegeben werden:

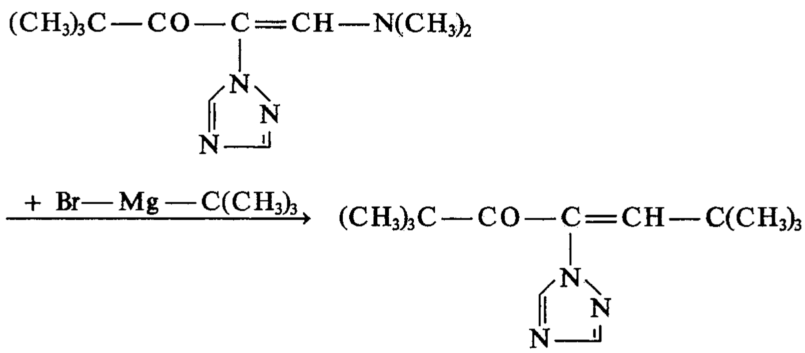


Verwendet man beispielsweise 2,2,6,6-Tetramethyl-4-(1,2,4-triazol-1-yl)-3-hepten-5-on und Natriumborhydrid als Ausgangsstoffe, so kann der Reaktionsablauf der erfindungsgemäßen Reduktion durch das folgende Formelschema wiedergegeben werden:

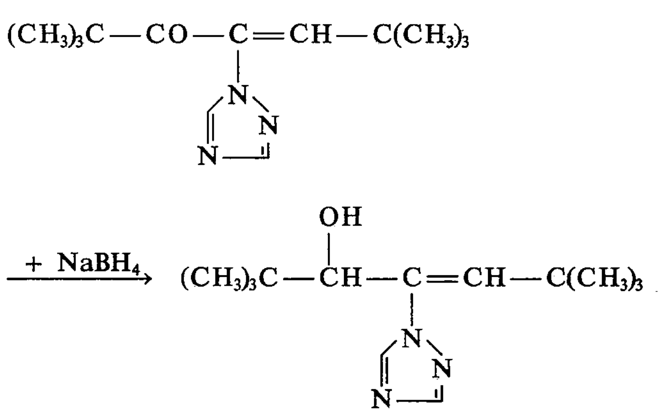


Die als Ausgangsstoffe für das erfindungsgemäße Verfahren zu verwendenden Ketoenamine sind durch die Formel (II) allgemein definiert. In dieser Formel steht $R^1$ vorzugsweise für diejenigen Reste, die bereits im Zusammenhang mit der Beschreibung der erfindungsgemäßen Stoffe der Formel (I) vorzugsweise für diesen Substituenten genannt wurden. $R^3$ und $R^4$ sind gleich oder verschieden und stehen vorzugsweise für Alkyl mit 1 bis 4 Kohlenstoffatomen, insbesondere für Methyl.

Die Ketoenamine der Formel (II) sind noch nicht bekannt; sie sind jedoch Gegenstand einer eigenen älteren Anmeldung, die noch nicht veröffentlicht ist (vergleiche die deutsche Patentanmeldung P 3 000 643.0 vom 10. 1. 80 [LeA 20 084]). Die Ketoenamine der Formel (II) können nach den dort beschriebenen Verfahren erhalten werden, indem man Triazolylketone der Formel

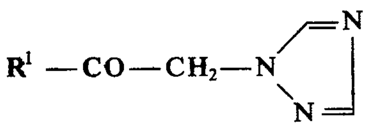

(IV)

in welcher

$R^1$ die oben angegebene Bedeutung hat,

mit Amidacetalen bzw. Aminalestern der Formeln

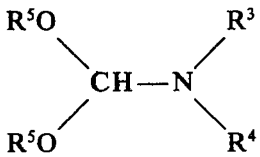

(Va)

bzw.

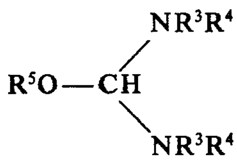

(Vb)

in welchen

$R^3$ und $R^4$ die oben angegebene Bedeutung haben und
$R^5$ für Alkyl mit 1 bis 4 Kohlenstoffatomen steht,

in an sich bekannter Art und Weise in Gegenwart eines inerten organischen Lösungsmittels, wie beispielsweise aromatische Kohlenwasserstoffe und wie insbesondere ein Überschuß an eingesetztem Amidacetal bzw. Aminalester der Formeln (Va) bzw. (Vb), in der Siedehitze umsetzt (vergleiche hierzu auch Chem. Ber. 101, 41—50 (1968); J. Org. Chem. 43, 4248—50 (1978) sowie die Herstellungsbeispiele).

Die Triazolyl-ketone der Formel (IV) sind bekannt (vergleiche z. B. DE-OS 2 431 407, DE-OS-2 610 022 und DE-OS-2 638 470), bzw. lassen sie sich nach üblichen Methoden herstellen, indem man die entsprechenden Halogen-ketone in Gegenwart eines Säurebinders mit 1,2,4-Triazol umsetzt.

Die Amidacetale bzw. Aminalester der Formeln (Va) bzw. (Vb) sind allgemein bekannte Verbindungen der organischen Chemie (vergleiche z. B. Chem. Ber. 101, 41-50 (1968) und J. Org. Chem. 4248-50 [1978]).

Die außerdem für die erfindungsgemäße Umsetzung als Ausgangsstoffe zu verwendenden magnesium-organischen Verbindungen sind durch die Formel (III) allgemein definiert. In dieser Formel steht $R^2$ vorzugsweise für diejenigen Reste, die bereits im Zusammenhang mit der Beschreibung der erfindungsgemäßen Stoffe der Formel (I) vorzugsweise für diesen Substituenten genannt wurden. Hal steht vorzugsweise für Chlor oder Brom.

Die magnesium-organischen Verbindungen der Formel (III) sind allgemein bekannte Verbindungen der organischen Chemie.

Als Lösungsmittel kommen für die erfindungsgemäße Umsetzung vorzugsweise inerte organische Lösungsmittel in reiner Form oder im Gemisch in Frage. Hierzu gehören vorzugsweise Ether, wie Diethylether, Methylethylether, Tetrahydrofuran oder Dioxan, aliphatische und aromatische Kohlenwasserstoffe, wie insbesondere Benzol, Toluol oder Xylol, sowie Hexamethylphosphorsäuretriamid.

Die Reaktionstemperaturen können bei der Durchführung des erfindungsgemäßen Verfahrens in einem größeren Bereich variiert werden. Im allgemeinen arbeitet man zwischen $-50$ und $+150°C$, vorzugsweise zwischen $-20$ und $+120°C$.

Die erfindungsgemäße Umsetzung kann in Gegenwart eines Inertgases, wie z. B. Stickstoff oder Helium durchgeführt werden.

Bei der Durchführung des erfindungsgemäßen Verfahrens setzt man auf 1 Mol Ketoenamin der Formel (II) vorzugsweise 1 bis 1,5 Mol an magnesium-organischer Verbindung der Formel (III) ein. Die Isolierung der Verbindungen der Formel (I) erfolgt in üblicher Weise.

Die erfindungsgemäße Reduktion erfolgt in üblicher Weise, z. B. durch Umsetzung mit komplexen Hydriden, gegebenenfalls in Gegenwart eines Verdünnungsmittels, oder durch Umsetzung mit Aluminiumisopropylat in Gegenwart eines Verdünnungsmittels.

Arbeitet man mit komplexen Hydriden, so kommen als Verdünnungsmittel für die erfindungsgemäße Umsetzung polare organische Lösungsmittel in Frage. Hierzu gehören vorzugsweise Alkohol, wie Methanol, Ethanol, Butanol, Isopropanol, und Ether, wie Diethylether oder Tetrahydrofuran. Die Reaktion wird im allgemeinen bei 0 bis 30° C, vorzugsweise bei 0 bis 20° C durchgeführt. Hierzu setzt man auf 1 Mol des Ketons der Formel (I) etwa 1 Mol eines komplexen Hydrids, wie Natriumhydrid oder Lithiumalanat, ein. Zur Isolierung der reduzierten Verbindungen der Formel (I) wird der Rückstand in verdünnter Salzsäure aufgenommen, anschließend alkalisch gestellt und mit einem organischen Lösungsmittel extrahiert. Die weitere Aufarbeitung erfolgt in üblicher Weise.

Arbeitet man mit Aluminiumisopropylat, so kommen als Verdünnungsmittel für die erfindungsgemäße Umsetzung bevorzugt Alkohole, wie Isopropanol, oder inerte Kohlenwasserstoffe, wie Benzol, in Frage. Die Reaktionstemperaturen können wiederum in einem größeren Bereich variiert werden; im allgemeinen arbeitet man zwischen 20 und 120° C, vorzugsweise bei 50 bis 100° C. Zur Durchführung der Reaktion setzt man auf 1 Mol des entsprechenden Ketons der Formel (I) etwa 1 bis 2 Mol Aluminiumisopropylat ein. Zur Isolierung der reduzierten Verbindungen der Formel (I) wird das überschüssige Lösungsmittel durch Destillation im Vakuum entfernt und die entstandene Aluminium-Verbindung mit verdünnter Schwefelsäure oder Natronlauge zersetzt. Die weitere Aufarbeitung erfolgt in üblicher Weise.

Zur Herstellung von physiologisch verträglichen Säureadditionssalzen der Verbindungen der Formel (I) kommen vorzugsweise folgende Säuren in Frage: Die Halogenwasserstoffsäuren, wie z. B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z. B. Essigsäure, Maleinsäure, Bernsteinsäure, Fumarsäure, Weinsäure, Zitronensäure, Salizylsäure, Sorbinsäure, Milchsäure, sowie Sulfonsäuren, wie z. B. p-Toluolsulfonsäure und 1,5-Naphthalindisulfonsäure. Die Säureadditions-Salze der Verbindungen der Formel (I) können in einfacher Weise nach üblichen Salzbildungsmethoden, z. B. durch Lösen einer Verbindung der Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Haupt- und der I. und II. sowie IV. bis VIII. Nebengruppe in Frage, wobei Kupfer, Zink, Mangan, Magnesium, Zinn, Eisen und Nickel beispielhaft genannt seien.

Als Anionen der Salze kommen solche in Betracht, die sich vorzugsweise von folgenden Säuren ableiten: Halogenwasserstoffsäuren, wie z. B. die Chlorwasserstoffsäure und die Bromwasserstoffsäure, ferner Phosphorsäure, Salpetersäure und Schwefelsäure.

Die Metallsalz-Komplexe von Verbindungen der Formel (I) können in einfacher Weise nach üblichen Verfahren erhalten werden, so z. B. durch Lösen des Metallsalzes in Alkohol, z. B. Ethanol und Hinzufügen zur Verbindung der Formel (I). Man kann Metallsalz-Komplexe in bekannter Weise, z. B. durch Abfiltrieren, Isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Omycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Getreidekrankheiten, wie Gersten- bzw. Getreidemehltau (Erysiphe graminis) und Streifenkrankheit der Gerste, von Venturia-Arten, wie gegen den Erreger des Apfelschorfs (Fusicladium dendriticum), von Erysiphe-Arten, wie gegen den Erreger des Gurkenmehltaus (Erysiphe cichoracearum); sowie zur Bekämpfung der Braunfäule der Tomate (Phytophthora infestans) eingesetzt werden. Besonders hervorzuheben ist, daß die erfindungsgemäßen Wirkstoffe nicht nur eine protektive Wirkung haben, sondern teilweise auch systemisch wirksam sind. So gelingt es, Pflanzen gegen Pilzbefall zu schützen, wenn man den Wirkstoff über den Boden und die Wurzel oder über das Saatgut den oberirdischen Teilen der Pflanze zuführt.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten

Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z. B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid; als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z. B. nichtionogene und anionische Emulgatoren, wie Polyoxyäthylen-Fettsäure-Ester, Polyoxyäthylen-Fettalkohol-Äther, z. B. Alkylarylpolyglykol-äther, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z. B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02%, am Wirkungsort erforderlich.

In entsprechenden Aufwandmengen zeigen die erfindungsgemäßen Stoffe auch eine pflanzenwachstumsregulierende bzw. herbizide Wirkung.

## Herstellungsbeispiele

### Beispiel 1

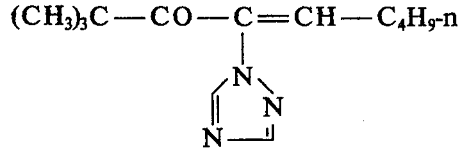

Zu 11,1 g (0,05 Mol) 4,4-Dimethyl-1-dimethylamino-2-(1,2,4-triazol-1-yl)-pent-1-en-3-on in 250 ml Ether gibt man bei −20°C innerhalb von 30 Minuten unter Inertgas 8,76 g (0,055 Mol) n-Butylmagnesiumbromid in 20 ml Ether. Nach beendeter Zugabe läßt man das Reaktionsgemisch innerhalb von ca. 2 Stunden auf Raumtemperatur erwärmen. Danach wird mit verdünnter Salzsäure

versetzt, die organische Phase wird abgetrennt, mit Wasser gewaschen, über Natriumsulfat getrocknet und eingeengt. Man erhält 11,2 g (95% der Theorie) 2,2-Dimethyl-4-(1,2,4-triazol-1-yl)-4-nonen-3-on mit einem Brechungsindex von $n_D^{20}$ =1,4858.

Herstellung des Ausgangsproduktes

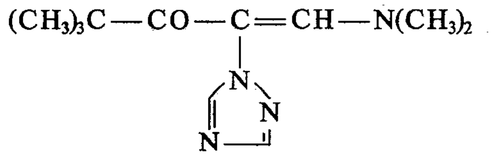

250,8 g (1,5 Mol) 3,3-Dimethyl-1-(1,2,4-triazol-1-yl)-butan-2-on werden mit 196 g (1,65 Mol) Dimethylformamiddimethylacetal 5 Stunden unter Rückfluß erhitzt. Danach wird das überschüssige Acetal abdestilliert. Man erhält 306 g (92% der Theorie) 4,4-Dimethyl-1-dimethylamino-2-(1,2,4-triazol-1-yl)-pent-1-en-3-on vom Brechungsindex $n_D^{20}$ =1,5310.

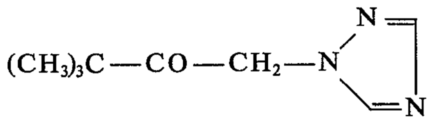

138 g (2 Mol) 1,2,4-Triazol werden bei Raumtemperatur portionsweise zu 276,4 g (2 Mol) gemahlenem Kaliumcarbonat und 259,2 g (2 Mol) α-Chlorpinakolin in 500 ml Aceton gegeben, wobei die Innentemperatur bis zur Siedehitze ansteigt. Man läßt 5 Stunden unter Rückfluß rühren und kühlt dann auf Raumtemperatur ab. Das Reaktionsgemisch wird filtriert und das Filtrat durch Abdestillieren des Lösungsmittels im Vakuum eingeengt. Der ölige Rückstand kristallisiert nach Zugabe von Benzin. Man erhält 240,8 g (72% der Theorie) 3,3-Dimethyl-1-(1,2,4-triazol-1-yl)-butan-2-on vom Schmelzpunkt 62—64° C.

Beispiel 2

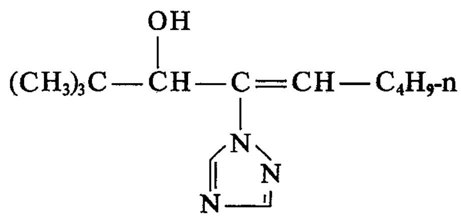

(Reduktion)

Zu 38,5 g (0,17 Mol) 2,2-Dimethyl-4-(1,2,4-triazol-1-yl)-5-nonen-3-on (Beispiel 1) in 160 ml Methanol werden bei 0° C 1,7 g (0,045 Mol) Natriumborhydrid, in 15 ml Wasser gelöst, getropft. Nach beendeter Zugabe läßt man das Reaktionsgemisch innerhalb von 2 Stunden auf Raumtemperatur erwärmen. Danach wird mit verdünnter Salzsäure ein pH-Wert von 6—7 eingestellt, die organische Phase wird abgetrennt, mit Wasser gewaschen, über Natriumsulfat getrocknet und eingeengt. Der Rückstand wird in Chloroform aufgenommen und erneut eingeengt. Man erhält 34,6 g (89% der Theorie) 2,2-Dimethyl-4-(1,2,4-triazol-1-yl)-4-nonen-3-ol vom Brechungsindex $n_D^{20}$ =1,4906.

Analog werden die folgenden Verbindungen der allgemeinen Formel (I)

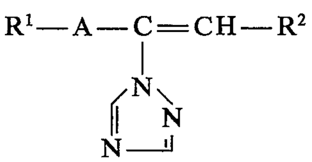

(I)

erhalten:

Fortsetzung

| Bsp. Nr. | $R^1$ | $R^2$ | A | Schmelzpunkt (°C) bzw. Brechungsindex ($n_D^{20}$) |
|---|---|---|---|---|
| 3 | $(CH_3)_3C$— | —$CH_2$— 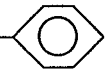 | CO | 1,5353 |
| 4 | $(CH_3)_3C$— | —C≡C— 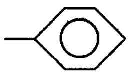 | CO | 1,6019 |
| 5 | $(CH_3)_3C$— | —$C_6H_{13}$-n | CO | 1,4935 |
| 6 | $(CH_3)_3C$— | —$C_9H_{19}$-n | CO | 1,4768 |
| 7 | $(CH_3)_3C$— | —$C_{10}H_{21}$-n | CO | 1,4788 |
| 8 | $(CH_3)_3C$— | —$C_{12}H_{25}$-n | CO | 1,4865 |
| 9 | $ClCH_2$<br>\|<br>$C(CH_3)_2$— | —$C_6H_{13}$-n | CO | 1,4964 |
| 10 | $(CH_3)_3C$— | —$CH_3$ | CO | 1,5025 |
| 11 | $(CH_3)_3C$— | —$C_2H_5$ | CO | 1,4915 |
| 12 | $(CH_3)_3C$— | —$C(CH_3)_3$ | CO | zähfl. Öl |
| 13 | $(CH_3)_3C$— | —$C_7H_{15}$-n | CO | 1,4708 |
| 14 | $ClCH_2$<br>\|<br>$C(CH_3)_2$— | —$C_8H_{17}$-n | CO | 1,4875 |
| 15 | $ClCH_2$<br>\|<br>$C(CH_3)_2$— | —$C_4H_9$-n | CO | 1,5022 |
| 16 | $(CH_3)_3C$— | 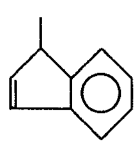 | CO | 110–13 |
| 17 | $(CH_3)_3C$— | 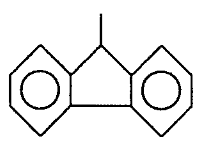 | CO | 176–78 |
| 18 | $(CH_3)_3C$— | —CH(CN)— 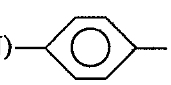 —Cl | CO | 126–27 |
| 19 | $ClCH_2$<br>\|<br>$C(CH_3)_2$— | —$C_7H_{15}$-n | CO | 1,4950 |
| 20 | $FCH_2$—$C(CH_3)_2$— | —$CH_3$ | CO | 1,5580 |
| 21 | $FCH_2$—$C(CH_3)_2$— | —$C_7H_{15}$-n | CO | 1,4764 |

Fortsetzung

| Bsp. Nr. | $R^1$ | $R^2$ | A | Schmelzpunkt (°C) bzw. Brechungsindex $(n_D^{20})$ |
|---|---|---|---|---|
| 22 | $Cl-CH_2$<br>\|<br>$C(CH_3)_2-$ | $-C_2H_5$ | CO | zähfl. Öl |
| 23 | $(CH_3)_3C-$ | 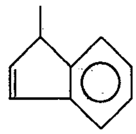 | CH(OH) | 74–80 |
| 24 | $(CH_3)_3C-$ | $-CH_3$ | CH(OH) | 1,4975 |
| 25 | $(CH_3)_3C-$ | $-C_7H_{15}$-n | CH(OH) | 1,4767 |
| 26 | $(CH_3)_3C-$ | $-C(CH_3)_3$ | CH(OH) | 35–37 |
| 27 | $(CH_3)_3C-$ | $-CH_2-$ 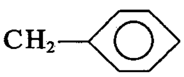 | CH(OH) | 107–09 |
| 28 | $(CH_3)_3C-$ | $-C\equiv C-$ 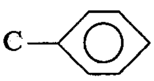 | CH(OH) | 88–91 |
| 29 | $(CH_3)_3C-$ | 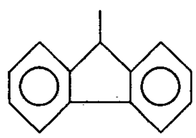 | CH(OH) | 210–14 |
| 30 | $(CH_3)_3C-$ | $-C_8H_{17}$-n | CH(OH) | 1,4809 |
| 31 | $FCH_2$<br>\|<br>$C(CH_3)_2-$ | $-C_7H_{15}$-n | CH(OH) | 1,4786 |
| 32 | $(CH_3)_3C-$ | $-CH_3$ | CO | 82 ($xCuCl_2$) |
| 33 | $(CH_3)_3C-$ | $-C_7H_{15}$-n | CO | zähfl. Öl ($xCuCl_2$) |
| 34 | $(CH_3)_3C-$ | $-C(CH_3)_3$ | CO | 73–77 ($xCuCl_2$) |
| 35 | $(CH_3)_3C-$ | 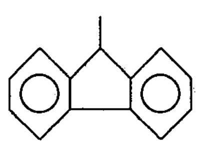 | CO | 148–50 ($xCuCl_2$) |
| 36 | $(CH_3)_3C-$ | 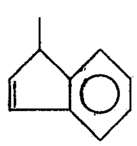 | CO | 116–18 ($xCuCl_2$) |
| 37 | $FCH_2$<br>\|<br>$C(CH_3)_2-$ | $-C_9H_{19}$-n | CO | 1,4679 |

Fortsetzung

| Bsp. Nr. | $R^1$ | $R^2$ | A | Schmelzpunkt (°C) bzw. Brechungsindex ($n_D^{20}$) |
|---|---|---|---|---|
| 38 | $(CH_3)_3C—$ | $C_{18}H_{37}$-n | CO | 1,4696 |
| 39 | $(CH_3)_3C—$ | 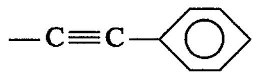 | CO | 75–80 ($xCuCl_2$) |
| 40 | $(CH_3)_3C—$ | $—C_2H_5$ | CO | 70 ($xCuCl_2$) |
| 41 | $(CH_3)_3C—$ | $—C_{12}H_{25}$-n | CH(OH) | 20 |
| 42 | $(CH_3)_3C—$ | $—CH_2—CH(CH_3)_2$ | CO | 1,4861 |
| 43 | $(CH_3)_3C—$ | $—CH_2CH_2CH(CH_3)_2$ | CO | 1,4834 |
| 44 | $(CH_3)_3C—$ | $—CH_2—CH(CH_3)_2$ | CH(OH) | 1,4870 |
| 45 | $(CH_3)_3C—$ | $—CH_2CH_2CH(CH_3)_2$ | CH(OH) | 1,4850 |

## Anwendungsbeispiele

In den nachfolgenden Beispielen werden die nachstehend angegebenen Verbindungen als Vergleichssubstanzen eingesetzt:

(A) 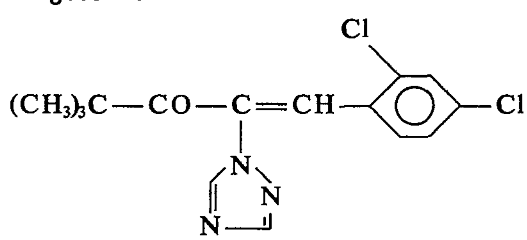

(B) 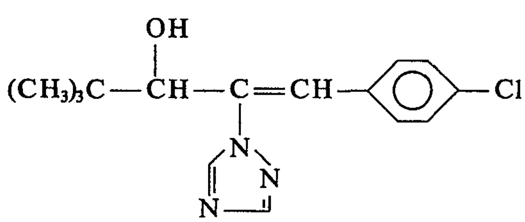

(C) 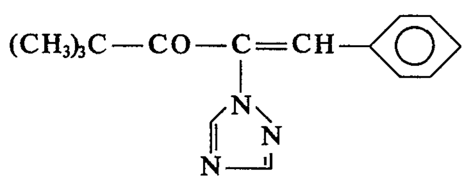

## Beispiel

Erysiphe-Test (Gerste) /protektiv/

Lösungsmittel: 100 Gewichtsteile Dimethylformamid
Emulgator: 0,25 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f. sp. hordei bestäubt.

Die Pflanzen werden in einem Gewächshaus bei einer Temperatur von ca. 20° C und einer relativen Luftfeuchtigkeit von ca. 80% aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z. B. die Verbindungen gemäß folgender Herstellungsbeispiele: 3, 13, 16, 4, 34, 2, 24, 27 und 26.

Tabelle A

Erysiphe-Test (Gerste)/protektiv

| Wirkstoff | Wirkstoffkonzentration in der Spritzbrühe in Gew.-% | Krankheitsbefall in % der unbehandelten Kontrolle |
|---|---|---|
| 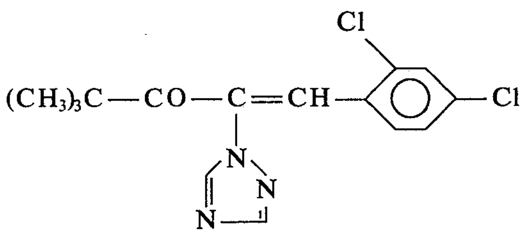 (A) (bekannt) | 0,025 | 100 |
| 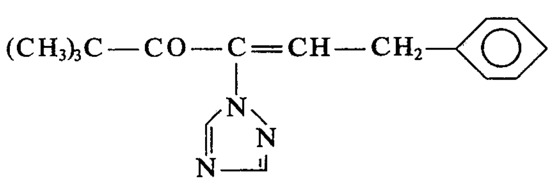 (3) | 0,025 | 0,0 |
| 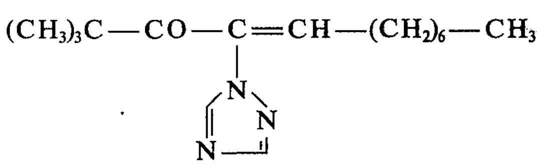 (13) | 0,025 | 25,0 |
| 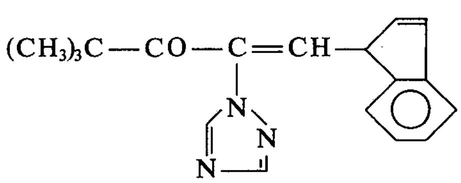 (16) | 0,025 | 21,3 |
| 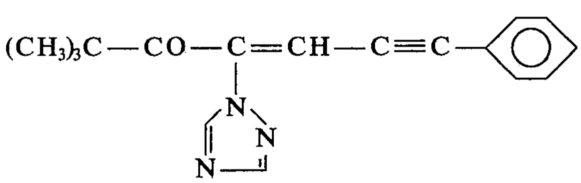 (4) | 0,025 | 0,0 |
| 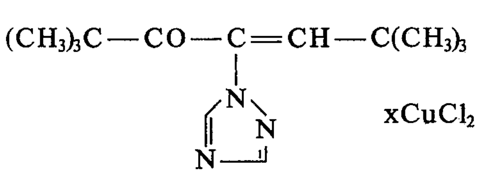 xCuCl$_2$ (34) | 0,025 | 31,3 |

Fortsetzung

| Wirkstoff | Wirkstoffkonzentration in der Spritzbrühe in Gew.-% | Krankheitsbefall in % der unbehandelten Kontrolle |
|---|---|---|
| 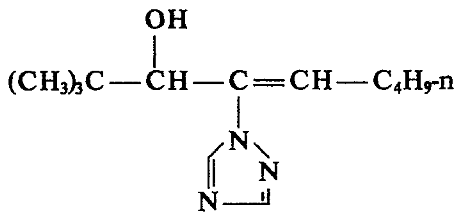 (2) | 0,025 | 0,0 |
| 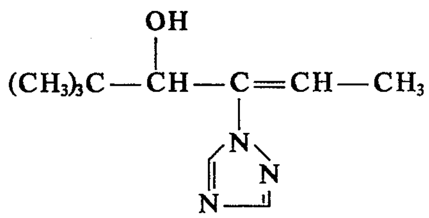 (24) | 0,025 | 33,8 |
| 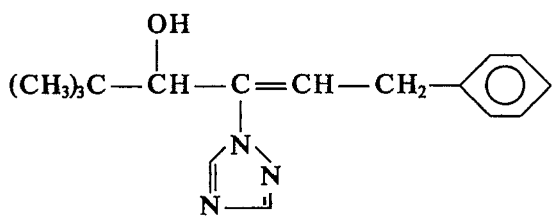 (27) | 0,025 | 0,0 |
| 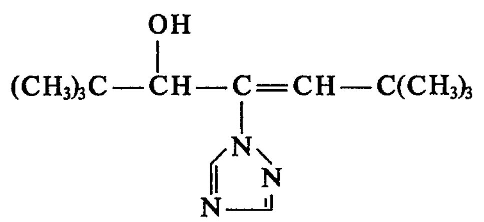 (26) | 0,025 | 21,3 |

Beispiel B

Saatgutbeizmittel-Test/Streifenkrankheit der Gerste (samenbürtige Mykose)

Zur Herstellung eines zweckmäßigen Trockenbeizmittels verstreckt man den Wirkstoff mit einem Gemisch aus gleichen Gewichtsteilen Talkum und Kieselgur zu einer feinpulverigen Mischung mit der gewünschten Wirkstoffkonzentration. Zur Beizung schüttelt man Gerstensaatgut, das durch Drechslera graminea (Syn. Helminthosporium gramineum) natürlich verseucht ist, mit dem Beizmittel in einer verschlossenen Glasflasche. Das Saatgut setzt man auf feuchten Filterscheiben in verschlossenen Petrischalen im Kühlschrank 10 Tage lang einer Temperatur von 4°C aus. Dabei wird die Keimung der Gerste und gegebenenfalls auch der Pilzsporen eingeleitet. Anschließend sät man die vorgekeimte Gerste mit 2 × 50 Korn 3 cm tief in Frühstorfer Einheitserde und kultiviert sie im Gewächshaus bei Temperaturen um 18°C in Saatkästen, die täglich 16 Stunden dem Licht ausgesetzt werden. Innerhalb von 3 bis 4 Wochen bilden sich die typischen Symptome der Streifenkrankheit aus.

Nach dieser Zeit bestimmt man die Anzahl der kranken Pflanzen in Prozent der insgesamt aufgelaufenen Pflanzen. Der Wirkstoff ist um so wirksamer je weniger Pflanzen erkrankt sind.

Bei diesem Test zeigen z. B. die folgenden Verbindungen eine sehr gute Wirkung, die derjenigen der aus dem Stand der Technik bekannten Verbindung (B) überlegen ist:

Verbindung gemäß Herstellungsbeispiel 2.

Tabelle B

Saatgutbeizmittel-Test/Streifenkrankheit der Gerste

| Wirkstoff | Wirkstoff-konzentration im Beizmittel in % | Beizmittel-aufwand-menge in g/kg Saatgut | Anzahl streifenkranker Pflanzen in % der insgesamt aufgelaufenen Pflanzen |
|---|---|---|---|
| ungebeizt | – | – | 42,1 |
| 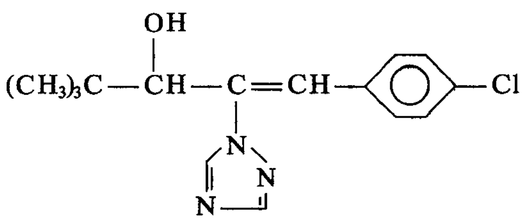 (B) (bekannt) | 10 | 2 | 45,3 |
| 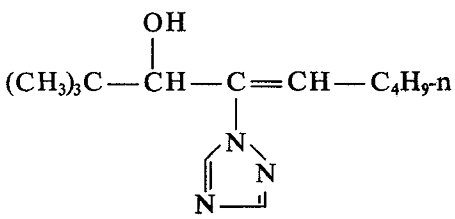 (2) | 10 | 2 | 2,1 |

Beispiel C

Fusicladium-Test (Apfel)/protektiv

Lösungsmittel: 4,7 Gewichtsteile Aceton
Emulgator: 0,3 Gewichtsteile Alkylarylpolyglykolether
Wasser: 95,0 Gewichtsteile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Apfelsämlinge, die sich im 4- bis 6-Blattstadium befinden, bis zur Tropfnässe. Die Pflanzen verbleiben 24 Stunden bei 20° C und einer relativen Luftfeuchtigkeit von 70% im Gewächshaus. Anschließend werden sie mit einer wäßrigen Konidiensuspension des Apfelschorferregers (Fusicladium dendriticum) inokuliert und 18 Stunden lang in einer Feuchtkammer bei 18 bis 20° C und 100% relativer Luftfeuchtigkeit inkubiert.

Die Pflanzen kommen dann erneut für 14 Tage ins Gewächshaus.

15 Tage nach der Inokulation wird der Befall der Sämlinge bestimmt. Die erhaltenen Boniturwerte werden in Prozent Befall umgerechnet. 0% bedeutet keinen Befall, 100% bedeutet, daß die Pflanzen vollständig befallen sind.

Bei diesem Test zeigen z. B. die folgenden Verbindungen eine sehr gute Wirkung, die derjenigen der aus dem Stand der Technik bekannten Verbindung (C) überlegen ist:

Verbindungen gemäß Herstellungsbeispielen 27, 26, 36, 34, 33, 32, 13 und 12.

**Tabelle C**

**Fusicladium-Test(Apfel)/protektiv**

| Wirkstoff | Befall in % bei einer Wirkstoff-konzentration von 0,01 % |
|---|---|
| 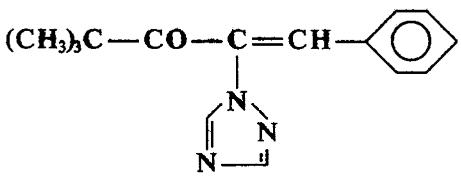 (C) (bekannt) | 77 |
| 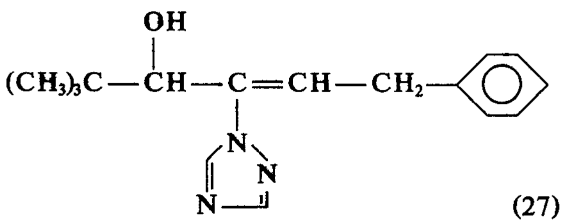 (27) | 10 |
| 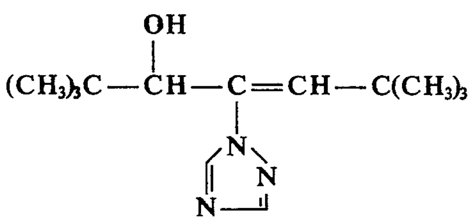 (26) | 21 |
| 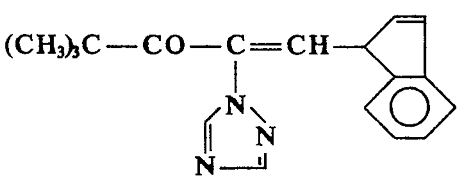 (36) x $CuCl_2$ | 20 |
| 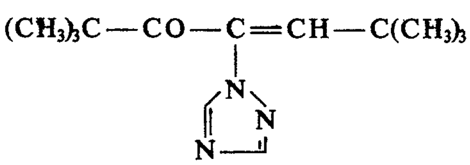 (34) x $CuCl_2$ | 11 |
| 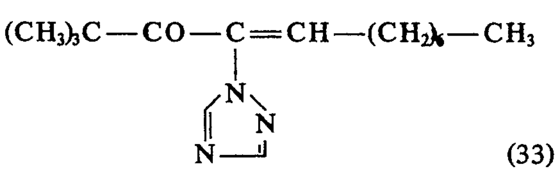 (33) x $CuCl_2$ | 2 |
| 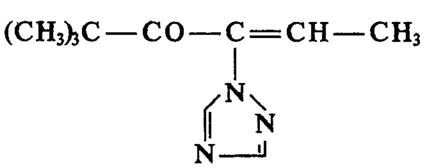 (32) x $CuCl_2$ | 12 |

Fortsetzung

| Wirkstoff | Befall in % bei einer Wirkstoff-konzentration von 0,01 % |
|---|---|
| 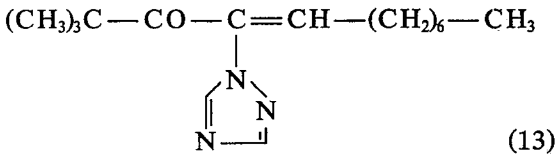 (13) | 20 |
| 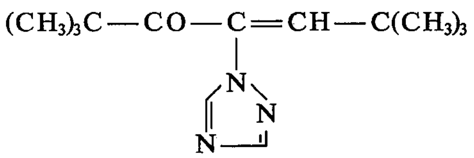 (12) | 35 |

**Patentansprüche**

1. Triazolyl-vinyl-ketone und -carbinole der allgemeinen Formel

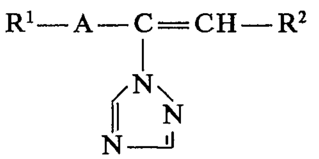

(I)

in welcher

A für die Ketogruppe oder eine CH(OH)-Gruppierung steht,
$R^1$ für Alkyl mit 1 bis 6 Kohlenstoffatomen oder für Halogenalkyl mit 1 bis 4 Kohlenstoffatomen und 1 bis 5 Halogenatomen steht,
$R^2$ für primäres oder tertiäres Alkyl mit 1 bis 29 Kohlenstoffatomen, für Halogenalkyl mit 1 bis 18 Kohlenstoff- und 1 bis 5 Halogenatomen steht, weiterhin für Alkoxyalkyl und Alkylmercaptoalkyl mit jeweils 1 bis 4 Kohlenstoffatomen in jedem Alkylteil steht, für Dialkylaminoalkyl mit 1 bis 4 Kohlenstoffatomen in den Alkylresten der Aminogruppe und 1 bis 18 Kohlenstoffatomen im Alkylteil steht, sodann für Hydroxylalkyl mit 1 bis 18 Kohlenstoffatomen steht, für Alkenyl, Alkinyl und Alkeninyl mit jeweils bis zu 6 Kohlenstoffatomen steht, wobei die drei letztgenannten Reste substituiert sein können durch Hydroxy, Alkoxy mit 1 bis 4 Kohlenstoffatomen und durch Phenyl, wobei letzteres selbst noch substituiert sein kann durch Halogen, durch Alkyl, Alkoxy und Alkylthio mit jeweils 1 bis 4 Kohlenstoffatomen und durch Halogenalkoxy mit 1 bis 4 Kohlenstoff- und 1 bis 5 Halogenatomen, und außerdem noch für Benzyl steht, welches im Phenylteil durch die weiter oben genannten Phenylsubstituenten und im Alkylteil durch Cyano substituiert sein kann, und schließlich noch für die Indenyl-, die Fluorenyl-, die Diphenylmethyl- und die Triphenylmethyl-Gruppe steht,

und deren physiologisch verträglichen Säureadditions-Salze sowie Metallsalz-Komplexe.

2. Verfahren zur Herstellung von Triazolyl-vinylketonen und -carbinolen der allgemeinen Formel

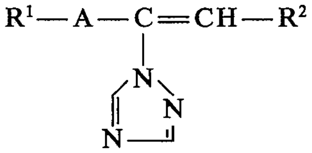

(I)

in welcher

A für die Ketogruppe oder eine CH(OH)-Gruppierung steht,
$R^1$ für Alkyl mit 1 bis 6 Kohlenstoffatomen oder für Halogenalkyl mit 1 bis 4 Kohlenstoffatomen und 1 bis 5 Halogenatomen steht,
$R^2$ für primäres oder tertiäres Alkyl mit 1 bis 29 Kohlenstoffatomen, für Halogenalkyl mit 1 bis 18 Kohlenstoff- und 1 bis 5 Halogenatomen steht, weiterhin für Alkoxyalkyl und Alkylmercaptoalkyl

mit jeweils 1 bis 4 Kohlenstoffatomen in jedem Alkylteil steht, für Dialkylaminoalkyl mit 1 bis 4 Kohlenstoffatomen in den Alkylresten der Aminogruppe und 1 bis 18 Kohlenstoffatomen im Alkylteil steht, sodann für Hydroxyalkyl mit 1 bis 18 Kohlenstoffatomen steht, für Alkenyl, Alkinyl und Alkeninyl mit jeweils bis zu 6 Kohlenstoffatomen steht, wobei die drei letztgenannten Reste substituiert sein können durch Hydroxy, Alkoxy mit 1 bis 4 Kohlenstoffatomen und durch Phenyl, wobei letzteres selbst noch substituiert sein kann durch Halogen, durch Alkyl, Alkoxy und Alkylthio mit jeweils 1 bis 4 Kohlenstoffatomen und durch Halogenalkoxy mit 1 bis 4 Kohlenstoff- und 1 bis 5 Halogenatomen, und außerdem noch für Benzyl steht, welches im Phenylteil durch die weiter oben genannten Phenylsubstituenten und im Alkylteil durch Cyano substituiert sein kann, und schließlich noch für die Indenyl-, die Fluorenyl-, die Diphenylmethyl- und die Triphenylmethyl-Gruppe steht,

dadurch gekennzeichnet, daß man Ketoenamine der Formel

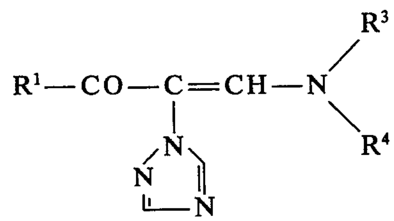

(II)

in welcher

| | |
|---|---|
| $R^1$ | die oben angegebene Bedeutung hat und |
| $R^3$ und $R^4$ | gleich oder verschieden sind und für Alkyl mit 1 bis 4 Kohlenstoffatomen stehen, |

mit magnesium-organischen Verbindungen der Formel

$$Hal-Mg-R^2 \qquad (III)$$

in welcher

| | |
|---|---|
| $R^2$ | die oben angegebene Bedeutung hat und |
| Hal | für Halogen steht, |

in Gegenwart eines Lösungsmittels umsetzt, und gegebenenfalls die entstehenden Keto-Derivate der Formel (I) in üblicher Weise reduziert, und weiterhin noch an die so erhaltenen Verbindungen gegebenenfalls anschließend eine Säure oder ein Metallsalz addiert.

3. Fungizide Mittel, gekennzeichnet durch einen Gehalt an mindestens einem Triazolyl-vinyl-keton oder -carbinol der Formel I in Ansprüchen 1 und 2.

4. Verwendung von Triazolyl-vinyl-ketonen oder -carbinolen der Formel I in Ansprüchen 1 und 2 zur Bekämpfung von Pilzen.

## Claims

1. Triazolyl-vinyl ketones and carbinols of the general formula

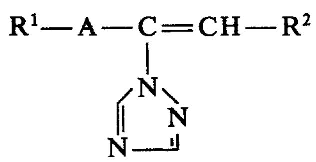

(I)

in which

A represents the keto group or a CH(OH) grouping,

$R^1$ represents alkyl with 1 to 6 carbon atoms or halogenoalkyl with 1 to 4 carbon atoms and 1 to 5 halogen atoms,

$R^2$ represents primary or tertiary alkyl with 1 to 29 carbon atoms, halogenalkyl with 1 to 18 carbon and 1 to 5 halogen atoms, furthermore represents alkoxyalkyl and alkylmercaptoalkyl with in each case 1 to 4 carbon atoms in each alkyl part, and represents dialkylaminoalkyl with 1 to 4 carbon atoms in the alkyl radicals of the amino group and 1 to 18 carbon atoms in the alkyl part, also represents hydroxyalkyl with 1 to 18 carbon atoms, and represents alkenyl, alkinyl and alkeninyl with in each case up to 6 carbon atoms, it being possible for the three last-mentioned radicals to be substituted

by hydroxyl, alkoxy with 1 to 4 carbon atoms and by phenyl, it being possible for the latter itself also to be substituted by halogen, by alkyl, alkoxy and alkylthio with in each case 1 to 4 carbon atoms and by halogenoalkoxy with 1 to 4 carbon and 1 to 5 halogen atoms, and in addition also represents benzyl which can be substituted in the phenyl part by the phenyl substituents mentioned further above and in the alkyl part by cyano, and finally also represents the indenyl, the fluorenyl, the diphenylmethyl and the triphenylmethyl group,

and physiologically acceptable acid addition salts and metal salt complexes thereof.

2. Process for the preparation of triazolyl-vinyl ketones and carbinols of the general formula

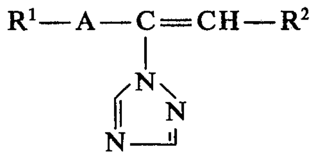

(I)

in which

A represents the keto group or a CH(OH) grouping,

$R^1$ represents alkyl with 1 to 6 carbon atoms or halogenoalkyl with 1 to 4 carbon atoms and 1 to 5 halogen atoms,

$R^2$ represents primary or tertiary alkyl with 1 to 29 carbon atoms, halogenoalkyl with 1 to 18 carbon and 1 to 5 halogen atoms, furthermore represents alkoxyalkyl and alkylmercaptoalkyl with in each case 1 to 4 carbon atoms in each alkyl part, and represents dialkylaminoalkyl with 1 to 4 carbon atoms in the alkyl radicals of the amino group and 1 to 18 carbon atoms in the alkyl part, also represents hydroxyalkyl with 1 to 18 carbon atoms, and represents alkenyl, alkinyl and alkeninyl with in each case up to 6 carbon atoms, it being possible for the three last-mentioned radicals to be substituted by hydroxyl, alkoxy with 1 to 4 carbon atoms and by phenyl, it being possible for the latter itself also to be substituted by halogen, by alkyl, alkoxy and alkylthio with in each case 1 to 4 carbon atoms and by halogenoalkoxy with 1 to 4 carbon and 1 to 5 halogen atoms, and in addition also represents benzyl which can be substituted in the phenyl part by the phenyl substituents mentioned further above and in the alkyl part by cyano, and finally also represents the indenyl, the fluorenyl, the diphenylmethyl and the triphenylmethyl group,

characterised in that ketoenamines of the formula

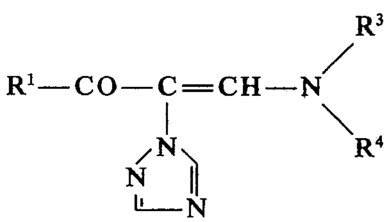

(II)

in which

$R^1$ has the meaning indicated above and

$R^3$ and $R^4$ are identical or different and represent alkyl with 1 to 4 carbon atoms,

are reacted with organo-magnesium compounds of the formula

$$Hal-Mg-R^2 \qquad (III)$$

in which

$R^2$ has the meaning indicated above and

Hal represents halogen,

in the presence of a solvent, and, if appropriate, the keto derivatives formed, of the formula (I), are reduced in the customary manner, and, furthermore, an acid or a metal salt is subsequently optionally also added on to the compounds thus obtained.

3. Fungicidal agents, characterised in that they contain at least one triazolyl-vinyl ketone or carbinol of the formula I in Claims 1 and 2.

4. Use of triazolyl-vinyl ketones or carbinols of the formula I in Claims 1 and 2 for combating fungi.

**Revendications**

1. Triazolyl-vinyl-cétones et triazolylvinyl-carbinols de formule générale:

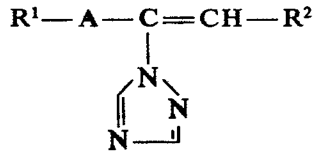

(I)

dans laquelle

A représente le groupe céto ou un groupement CH(OH),

$R^1$ représente un groupe alkyle contenant 1 à 6 atomes de carbone ou un groupe halogénalkyle contenant 1 à 4 atomes de carbone et 1 à 5 atomes d'halogènes,

$R^2$ représente un groupe alkyle primaire ou tertiaire contenant 1 à 29 atomes de carbone, un groupe halogénalkyle contenant 1 à 18 atomes de carbone et 1 à 5 atomes d'halogènes, de même qu'un groupe alcoxyalkyle et un groupe alkylmercaptoalkyle contenant chacun 1 à 4 atomes de carbone dans chaque fraction alkyle, un groupe dialkylaminoalkyle contenant 1 à 4 atomes de carbone dans les radicaux alkyle du groupe amino et 1 à 18 atomes de carbone dans la fraction alkyle, un groupe hydroxyalkyle contenant 1 à 18 atomes de carbone, un groupe alcényle, un groupe alcinyle et un groupe alcéninyle contenant chacun jusqu'à 6 atomes de carbone, les trois radicaux mentionnés en dernier lieu pouvant être substitués par un groupe hydroxy, par un groupe alcoxy contenant 1 à 4 atomes de carbone et par un groupe phényle, ce dernier lui-même pouvant encore être substitué par un atome d'halogène, par un groupe alkyle, par un groupe alcoxy et par un groupe alkylthio contenant chacun 1 à 4 atomes de carbone, de même que par un groupe halogénalcoxy contenant 1 à 4 atomes de carbone et 1 à 5 atomes d'halogènes, $R^2$ pouvant encore représenter, en outre, un groupe benzyle qui peut être substitué dans la fraction phényle par les autres substituants de phényle mentionnés ci-dessus et, dans la fraction alkyle, par un groupe cyano, $R^2$ pouvant enfin encore représenter le groupe indényle, le groupe fluorényle, le groupe diphénylméthyle et le groupe triphénylméthyle,

de même que leurs complexes de sels métalliques et leurs sels d'addition d'acide physiologiquement compatibles.

2. Procédé de préparation de triazolyl-vinyl-cétones et de triazolyl-vinyl-carbinols de formule générale:

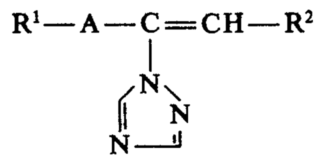

(I)

dans laquelle

A représente le groupe céto ou un groupement CH(OH),

$R^1$ représente un groupe alkyle contenant 1 à 6 atomes de carbone ou un groupe halogénalkyle contenant 1 à 4 atomes de carbone et 1 à 5 atomes d'halogènes,

$R^2$ représente un groupe alkyle primaire ou tertiaire contenant 1 à 29 atomes de carbone, un groupe halogénalkyle contenant 1 à 18 atomes de carbone et 1 à 5 atomes d'halogènes, de même qu'un groupe alcoxyalkyle et un groupe alkylmercaptoalkyle contenant chacun 1 à 4 atomes de carbone dans chaque fraction alkyle, un groupe dialkylaminoalkyle contenant 1 à 4 atomes de carbone dans les radicaux alkyle du groupe amino et 1 à 18 atomes de carbone dans la fraction alkyle, un groupe hydroxyalkyle contenant 1 à 18 atomes de carbone, un groupe alcényle, un groupe alcinyle et un groupe alcéninyle contenant chacun jusqu'à 6 atomes de carbone, les trois radicaux mentionnés en dernier lieu pouvant être substitués par un groupe hydroxy, par un groupe alcoxy contenant 1 à 4 atomes de carbone et par un groupe phényle, ce dernier lui-même pouvant encore être substitué par un atome d'halogène, par un groupe alkyle, par un groupe alcoxy et par un groupe alkylthio contenant chacun 1 à 4 atomes de carbone, de même que par un groupe halogénalcoxy contenant 1 à 4 atomes de carbone et 1 à 5 atomes d'halogènes, $R^2$ pouvant encore représenter, en outre, un groupe benzyle qui peut être substitué dans la fraction phényle par les autres substituants de phényle mentionnés ci-dessus et, dans la fraction alkyle, par un groupe cyano, $R^2$ pouvant enfin encore représenter le groupe indényle, le groupe fluorényle, le groupe diphénylméthyle et le groupe triphénylméthyle,

caractérisé en ce qu'on fait réagir des céto-énamines de formule:

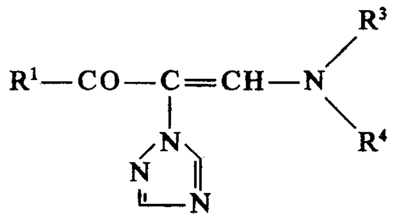 (II)

dans laquelle

$R^1$ a la signification indiquée ci-dessus et
$R^3$ et $R^4$ sont identiques ou différents et représentent chacun un groupe alkyle contenant 1 à 4 atomes de carbone,

avec des composés organiques de magnésium de formule:

$$Hal-Mg-R^2 \qquad (III)$$

dans laquelle

$R^2$ a la signification indiquée ci-dessus, et
Hal représente un atome d'halogène,

en présence d'un solvant, et on réduit éventuellement les céto-dérivés obtenus de formule (I) de la manière habituelle tandis que, en outre, aux composés ainsi obtenus, on ajoute encore éventuellement ensuite un acide ou un sel d'un métal.

3. Agents fongicides caractérisés en ce qu'ils contiennent au moins une triazolyl-vinylcétone ou un triazolyl-vinyl-carbinol de formule I suivant les revendications 1 et 2.

4. Utilisation de triazolyl-vinyl-cétones ou de triazolyl-vinyl-carbinols de formule I suivant les revendications 1 et 2 pour combattre les champignons.